# FASCICULE DE BREVET EUROPEEN

(11) **EP 2 776 598 B1**
(45) Date de publication et mention de la délivrance du brevet: **03.02.2016**
(21) Numéro de dépôt: 12781109.9
(22) Date de dépôt: 07.11.2012
(51) Int. Cl.: C22C 14/00, C22F 1/18, A61L 27/50, A61L 27/06

(54) **PROCEDE DE TRAITEMENT THERMOMECANIQUE D'UN ALLIAGE DE TITANE, ALLIAGE ET PROTHESE AINSI OBTENUS**
VERFAHREN ZUR THERMOMECHANISCHEN BEHANDLUNG EINER TITANLEGIERUNG, DARAUS RESULTIERENDE LEGIERUNG UND PROTHESE
METHOD FOR THE THERMOMECHANICAL TREATMENT OF A TITANIUM ALLOY, AND RESULTING ALLOY AND PROSTHESIS

(30) Priorité: 10.11.2011 FR 1160228
(43) Date de publication de la demande: 17.09.2014
(73) Titulaire: Université de Lorraine, 54052 Nancy Cedex (FR); Ecole Nationale d'Ingenieurs de Metz (ENIM), 57078 Metz Cedex 3 (FR); Arts, 75013 Paris (FR); Institut National des Sciences Appliquées de Rennes, 35708 Rennes Cedex 7 (FR); Centre National de la Recherche Scientifique (CNRS), 75794 Paris Cedex 16 (FR)
(72) Inventeur: LAHEURTE, Pascal, 57600 Forbach (FR); PRIMA, Frédéric, 75015 Paris (FR); GLORIANT, Thierry, 35250 Mouaze (FR); ELMAY, Wafa, 57000 Metz (FR); EBERHARDT, André, 57070 Metz (FR); PATOOR, Etienne, 57050 Longevilles les Metz (FR)
(74) Mandataire: Novagraaf Technologies
(86) Numéro de dépôt international: PCT/EP2012/071963
(87) Numéro de publication internationale: WO 2013/068366

(56) Documents cités:
- EP-A1- 0 437 079
- HAO ET AL: "Elastic deformation behaviour of Ti-24Nb-4Zr-7.9Sn for biomedical applications", ACTA BIOMATERIALIA, ELSEVIER, AMSTERDAM, NL, vol. 3, no. 2, 13 février 2007 (2007-02-13), pages 277-286, XP005886205, ISSN: 1742-7061, DOI: 10.1016/J.ACTBIO.2006.11.002
- SUN F ET AL: "Influence of a short thermal treatment on the superelastic properties of a titanium-based alloy", SCRIPTA MATERIALIA, ELSEVIER, AMSTERDAM, NL, vol. 63, no. 11, 1 novembre 2010 (2010-11-01), pages 1053-1056, XP027289858, ISSN: 1359-6462 [extrait le 2010-08-02]

## Description

### DOMAINE DE L'INVENTION

L'invention concerne un procédé de traitement thermomécanique d'un alliage de titane afin d'obtenir un faible module d'élasticité et une grande résistance mécanique. Elle concerne également un alliage ainsi obtenu et une prothèse réalisée dans cet alliage, en particulier un implant dentaire.

### TECHNIQUE ANTÉRIEURE

Dans la médecine actuelle, des prothèses sont utilisées afin de remplacer des fonctions qui ne sont plus ou mal réalisées par le corps d'un patient. On réalise ainsi des prothèses qui remplacent une articulation, telle que la hanche ou le genou. On fixe aussi des éléments qui viennent soutenir le squelette, de manière permanente ou temporaire. Dans certaines configurations, la prothèse est réalisée en plusieurs parties, dont certaines ressemblent à des vis qui sont logées dans de l'os et qui permet la liaison mécanique entre l'os et une autre partie de la prothèse. On réalise aussi des implants dentaires qui sont insérés dans l'os de la mâchoire à la place d'une dent et qui reçoivent des prothèses de dent. Dans la suite de la description le terme de prothèse désigne également les implants dentaires.

On réalise fréquemment les prothèses en métal afin qu'elles aient les bonnes propriétés de résistance mécanique. Actuellement, les prothèses métalliques sont réalisées à partir d'alliages Cr-Co, d'acier inoxydable, de titane, d'alliages de titane biphasés α+β (TA6V ELI) et d'alliages Ni-Ti (Nickel-Titane). Le titane et ses alliages sont majoritairement utilisés pour leurs propriétés mécaniques et leur biocompatibilité. Cette biocompatibilité n'est pas toujours totale car certains éléments de ces alliages, tels que le nickel, le vanadium et l'aluminium, sont considérés comme potentiellement toxiques.

L'amélioration de la biocompatibilité chimique passe par une nouvelle formulation qui ne comporte que des éléments biocompatibles chimiquement. Par exemple, les éléments choisis parmi le tantale, le zirconium, le niobium et le titane sont considérés comme ne présentant pas de problèmes de biocompatibilité chimique.

D'autres considérations doivent également être prises en compte pour la réalisation de bonnes prothèses. Dans le cas où la prothèse doit s'intégrer à un os, on constate que le module d'élasticité du matériau de la prothèse a un rôle déterminant dans la réussite de l'intégration de la prothèse. En effet, lorsque la prothèse a une rigidité supérieure à celle de l'os, il se produit un phénomène de déviation des contraintes qui diminue les contraintes dans certaines zones, et les concentre dans d'autres. Dans les cas limites, l'os se détériore à l'interface avec la prothèse et l'ancrage de celle-ci n'est pas réalisé correctement.

La biocompatibilité mécanique consiste à adapter la raideur de l'implant à celle de l'os afin d'assurer un transfert de charge mécanique plus homogène favorable à l'ostéointégration. Le module d'élasticité des alliages de titane, bien que plus faible que celui de l'acier reste encore élevé (110 GPa) si on le compare à celui des tissus biologiques hôtes, de l'ordre de 20 GPa pour l'os.

Parmi les autres propriétés intéressantes des matériaux pour la réalisation de prothèse, on porte une attention particulière à la super-élasticité et à la pseudo-élasticité. Des alliages de Ni-Ti ou de titane β sont particulièrement utilisés pour leurs propriétés spécifiques de super-élasticité ou pseudo-élasticité, qui leur confère une capacité à retrouver leur forme initiale, ou au moins à avoir une forte déformation recouvrable après avoir subi une déformation importante sous l'effet d'une contrainte, contrairement à l'acier qui, à déformation initiale égale, subirait une déformation permanente et aurait une déformation recouvrable faible.

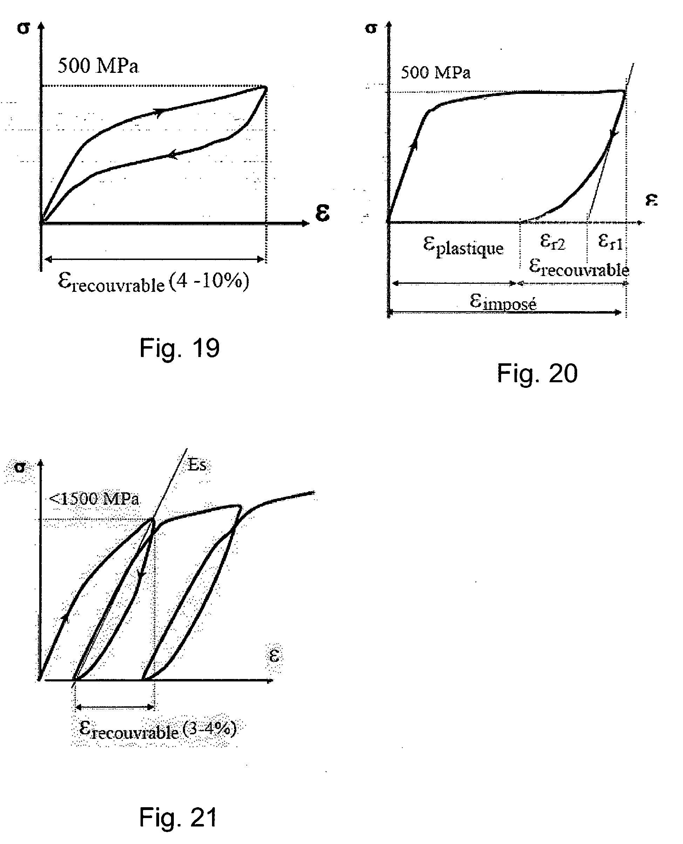

La super-élasticité se caractérise par une courbe de déformation en fonction de la contrainte telle que représentée figure 19, et résulte d'une transformation de phase à l'état solide appelée transformation martensitique : sous l'effet d'une contrainte, un échantillon de cet alliage, qui est au repos à l'état d'austénite, se transforme partiellement en martensite. Lorsque l'application de la contrainte cesse, on observe un retour à l'état austénitique par transformation inverse et l'échantillon retrouve sa dimension initiale, avec toutefois un effet d'hystérésis qui déplace la courbe de décharge nettement sous la courbe de charge. Ainsi, alors que des alliages métalliques classiques possèdent une déformation élastique maximum d'environ 0,2 %, les alliages ayant des propriétés super-élastiques peuvent présenter une déformation réversible recouvrable ε_{recouvrable} de 10%.

L'effet pseudo-élastique est illustré sur la figure 20. On voit que, après une forte déformation imposée ε_{imposée}, le relâchement de la contrainte provoque un retour selon une courbe de décharge incurvée, laissant subsister une certaine déformation plastique ε_{plastique}. Cette courbe de décharge spécifique peut s'expliquer par une combinaison d'une partie de retour de type purement élastique, avec une autre partie de type superélastique ou pseudoélastique due à la réversibilité de la transformation martensitique ou à la réorientation de variantes de martensite, comme cela est connu par exemple du document T.W. Duerig, R. Zadno An Enginer's Perspectives of Pseodoelasticity, Engineering aspect of SMA, Edt. T.W Duerig, Butterworth-Heinemann Publishers,p369, London, 1990. La déformation recouvrable ε_{recouvrable}, qui est l'amplitude de la déformation pendant la décharge, résulte de la somme du retour élastique εᵣ₁ et du retour super élastique εᵣ₂, et peut être de l'ordre de 3 à 4 %.

Sur la figure 21, on a représenté les conséquences de l'effet pseudoélastique lorsqu'un échantillon en un alliage possédant ces propriétés est soumis à une succession de déformations imposées et de relâchements de contrainte. On parle alors de pseudo-élasticité linéaire pour désigner le retour selon la courbe incurvée mentionnée ci-dessus, qui se répète de manière quasi identique suite à chaque déformation imposée, dans la limite bien sûr d'une déformation conduisant à la rupture.

Il a été proposé dans le document US 6,752,882 d'utiliser un alliage binaire de titane et de niobium pour ses propriétés de biocompatibilité. Le module d'Young de cet alliage est d'au moins 60 GPa, ce qui est encore trop éloigné de celui de l'os.

On connaît par le document US 7,722,805 B2 des alliages de titane, de niobium et de zirconium conçus pour améliorer la biocompatibilité. Un faible module d'Young est recherché par une optimisation de la composition chimique et par des traitements thermiques. En particulier, il est montré que le module d'Young peut être abaissé en optimisant la composition chimique et par un traitement thermique de trempe à l'eau. Un traitement de revenu implique une remontée de la valeur du module d'Young.

Le document WO 2007/137772 montre un alliage de titane composé de 28% en masse de niobium, 1% en masse de fer et 0,5% en masse de silicium. Un lingot cylindrique est forgé à 850 °C, puis maintenu à 900°C pendant une heure pour former une phase β, et trempé à l'eau. Ensuite une déformation à froid de 60 à 95% est donnée à l'échantillon. On obtient un matériau ayant un module d'Young de 37,5 GPa, une résistance élastique de 998 MPa et un allongement à la rupture de 10,7%.

EP0437079 divulgue un procédé de traitement d'un alliage de titane comportant une déformation à froid d'un échantillon de alliage (wt.%): titane, 10-20% Nb ou 35-50% Nb, et 0-20% Zr, et puis un traitement de vieillissement.

### OBJECTIFS DE L'INVENTION

L'invention vise à fournir un procédé de traitement thermomécanique d'un alliage en titane permettant d'obtenir un très bas module d'Young et une haute résistance mécanique, avec une bonne biocompatibilité chimique et mécanique. Elle vise également à obtenir des prothèses réalisées avec un tel alliage.

### EXPOSÉ DE L'INVENTION

Avec ces objectifs en vue, l'invention a pour objet un procédé de traitement thermomécanique d'un alliage de titane comportant entre 23 et 27% de niobium, entre 0 et 10% de zirconium, et entre 0 et 1% d'oxygène, d'azote et/ou de silicium, les taux étant exprimés en proportion atomique, procédé selon lequel on procède aux étapes suivantes :
a) une montée en température d'un échantillon de l'alliage à une température supérieure à 900°C ;
b) une trempe ;
c) une déformation à froid supérieure à une déformation vraie de 3 ;
d) un traitement de vieillissement à une température comprise entre 200 et 600°C, la durée du traitement de vieillissement étant comprise entre 10 secondes et 10 minutes.

On constate qu'avec un tel procédé, on obtient un alliage de titane ayant toutes les qualités souhaitées, en particulier un bas module d'Young, une haute résistance mécanique et une bonne biocompatibilité, à la fois chimique et mécanique. Plus particulièrement, on constate qu'un tel alliage est favorablement sensible à une durée très courte de traitement de vieillissement, c'est-à-dire que le module d'Young est abaissé par ce traitement en même temps que la résistance mécanique est augmentée. Si la durée du traitement de vieillissement est augmentée, on obtient une augmentation du module d'Young. On constate également que cet alliage comporte d'excellentes propriétés de superélasticité. La combinaison des étapes a) et b) est appelée aussi recuit de mise en solution. Le recuit sert à transformer l'alliage en phase austénitique β. La trempe est un refroidissement thermique suffisamment rapide pour ne pas laisser de mécanisme de diffusion se mettre en place. La déformation à froid peut être obtenue par laminage, par estampage ou par étirage, en une ou plusieurs passes. La déformation vraie est égale au logarithme naturel du quotient de la dimension après déformation et de la dimension initiale.

De manière particulière, l'étape a) est précédée d'une étape d'homogénéisation à une température supérieure à 1200 °C pendant au moins 20 heures. La composition chimique est ainsi régulièrement répartie à l'intérieur de l'échantillon.

Selon des caractéristiques particulières du procédé :
- à l'étape c), la déformation est comprise entre 400 et 500% de la dimension finie ;
- à l'étape c), la déformation est réalisée uniquement en surface d'un échantillon ; une telle déformation est réalisée par exemple par un grenaillage ou par un microbillage.

L'invention a aussi pour objet un alliage de titane comportant entre 23 et 27% de niobium, entre 0 et 10% de zirconium, et entre 0 et 1% d'oxygène, d'azote et/ou de silicium, les taux étant exprimés en proportion atomique, l'alliage étant obtenu par le procédé tel que décrit précédemment et son module de Young étant inférieur à 45 GPa.

De manière particulière :
- le taux de niobium est compris entre 24 et 26% en proportion atomique ;
- le taux de zirconium est de 0%.

A titre d'exemple, différentes versions de l'alliage sont conformes à l'invention :
- le taux de niobium de l'alliage est de 24%, la structure cristallographique étant une phase β avec des traces de martensite α, et d'une phase ω après un traitement de vieillissement à 300°C.
- le taux de niobium est de 24%, la structure cristallographique comportant une phase β, de la martensite α et de la martensite α" après un traitement de vieillissement à 600°C.
- le taux de niobium est de 26%, la structure cristallographique comportant une phase β et de la martensite α".

L'invention a également pour objet une prothèse implantable dans un corps humain ou animal, caractérisée en ce qu'elle est réalisée dans un alliage tel que décrit précédemment. Une telle prothèse est par exemple un implant dentaire. Ce peut être aussi par exemple une agrafe, une vis, une endoprothèse vasculaire, une articulation ou un ressort.

### BRÈVE DESCRIPTION DES FIGURES

L'invention sera mieux comprise et d'autres particularités et avantages apparaîtront à la lecture de la description qui va suivre, la description faisant référence aux dessins annexés parmi lesquels :
- les figures 1 et 2 sont des diagrammes de diffraction de rayons X pour des échantillons recuits ;
- les figures 3 et 4 sont des vues au microscope optique des échantillons des figures 1 et 2 ;
- la figure 5 montre des courbes d'essai de traction-relâchement pour les échantillons des figures 1 et 2 ;
- les figures 6 et 7 sont des diagrammes de diffraction de rayons X pour des échantillons recuits et laminés à froid, puis ayant subi un traitement thermique de courte durée ;
- les figures 8 et 9 montrent des courbes d'essai de traction-relâchement pour des échantillons des figures 6 et 7 ;
- les figures 10 et 11 sont des diagrammes montrant respectivement le module d'Young et la déformation recouvrable pour les échantillons des figures 1, 2, 6 et 7 ;
- les figures 12 à 14 sont des diagrammes montrant respectivement l'évolution du module d'Young, de la résistance et de la déformation recouvrable en fonction de la durée du traitement thermique de vieillissement ;
- la figure 15 montre une courbe d'essai de traction-relâchement pour un échantillon ayant subi un traitement thermique de 5min, comparé à d'autres échantillons ;
- la figure 16 montre une courbe d'essai de traction-relâchement pour un échantillon comportant du zirconium ;
- la figure 17 montre un digramme représentant le niveau de contrainte pour un implant dentaire placé dans l'os d'une mâchoire ;
- la figure 18 montre un diagramme représentant le niveau de contrainte selon un rayon issu de la figure 17, pour différents modules d'Young correspondant à différents matériaux ;
- les figures 19 à 21 montrent des courbes d'essai de traction relâchement pour illustrer différents comportements typiques de matériaux super-élastiques ou pseudo-élastiques.

### DESCRIPTION DETAILLÉE

Dans tous les exemples qui suivent, le procédé d'élaboration de l'alliage commence par la préparation d'un lingot en mélangeant les métaux dans les proportions voulues et en les faisant fondre dans un four, par exemple un four à creuset froid chauffé par induction magnétique. Ceci garantit une grande pureté de l'alliage par absence de contact de la masse liquide avec le creuset. Après son moulage à une section de 20 x 20 mm environ, le lingot est homogénéisé à une température comprise entre 1200 et 1300 °C pendant environ 20 h. Après refroidissement, le lingot est laminé pour le ramener à une section de 10 x 10 mm. Il est ensuite monté à une température de 900 °C environ et y est maintenu pendant 1 heure, puis est trempé à l'eau. Compte-tenu de la faible section du lingot, la trempe est très rapide et elle peut être qualifiée d'hyper-trempe.

### Exemple 1

Deux échantillons sont préparés selon le procédé exposé précédemment, l'échantillon A1 comportant 24% de niobium en proportion atomique, le reste étant du titane, et l'échantillon B1 comportant 26% de niobium, le reste étant du titane également.

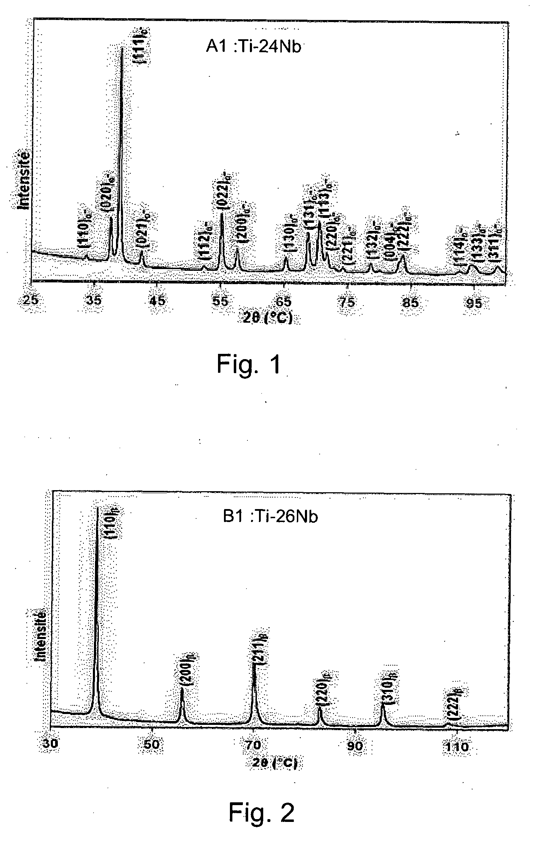

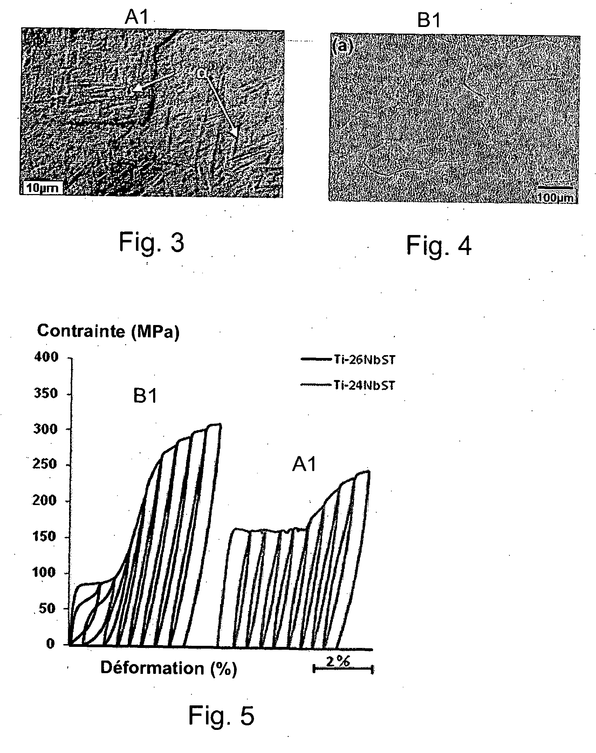

Une analyse par diffraction de rayons X est montrée sur les figures 1 et 2 respectivement. La figure 1 concerne l'échantillon A1 et montre distinctement des pics qui correspondent à une phase de martensite α" de structure orthorhombique, tandis que l'échantillon B1, sur la figure 2, ne présente que des pics correspondant à une unique phase β cubique centrée. La figure 3 montre une vue au microscope optique de l'échantillon A1, sur laquelle on observe clairement des variantes de martensite α". Deux morphologies caractéristiques dominantes sont observées au microscope électronique à transmission, à savoir une forme triangulaire et une forme en V. La figure 4, concernant l'échantillon B1, montre que l'échantillon est composé de grains équiaxiaux de phase β de dimension de l'ordre de 100 µm.

Des essais de caractérisation mécanique ont été conduits en effectuant des cycles de traction-relâchement avec des incréments de déformation constants. Les courbes reliant la déformation à la contrainte sont reportées sur la figure 5. Concernant l'échantillon B1, celui-ci montre un comportement superélastique avec une résistance à la rupture de 280 MPa. Ce comportement s'explique par le fait que, pour cet alliage, la température de transformation martensitique Ms (270K) est inférieure à la température ambiante. A température ambiante, l'alliage est composé de phase β, sous forme d'austénite. L'échantillon récupère tout son allongement jusqu'à une déformation de 1% grâce à la transformation de phase austénite/martensite pendant l'allongement du fait de la contrainte et à sa transformation inverse durant le déchargement. Lors de l'allongement de 1% à 2,5%, la transformation inverse martensite/austénite est partielle, ce qui explique les déformations résiduelles. Les changements de phase répétés austénite/martensite introduisent des défauts dans le matériau. Ces défauts sont responsables d'aspects d'allongements localisés et de la conservation de variantes de martensite. Concernant l'échantillon A1, la résistance à la rupture est de 240 MPa. Aucun comportement pseudo-élastique n'est observé au premier cycle de déformation. Ceci est corrélé au fait que la température de transformation martensitique Ms (340K) est supérieure à la température ambiante pour cette composition. Le comportement pseudo-élastique qui est observé ensuite pourrait s'expliquer par la réversibilité du mouvement des doubles frontières dans la martensite induite par la déformation.

### Exemple 2

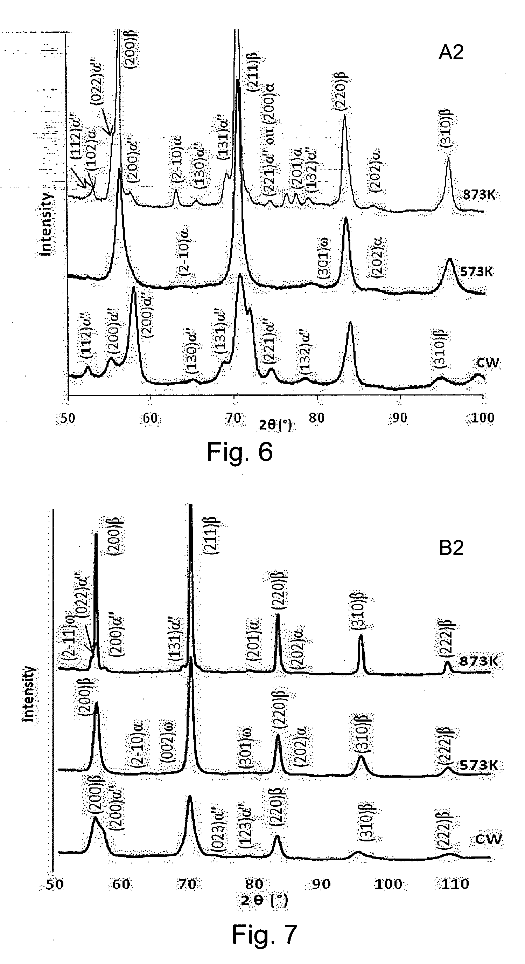

Des échantillons issus du procédé exposé dans l'exemple 1 subissent un laminage à froid sévère appliquant une déformation vraie supérieure à 3. Puis ils subissent un traitement thermique de vieillissement de courte durée de 10 min terminé par une trempe à l'eau. Deux niveaux de température ont été explorés : 573K et 873K (300 et 600 °C). La figure 6 montre trois diagrammes de diffraction de rayons X pour un échantillon A1 laminé et non vieilli (A2 CW), un échantillon A1 laminé et vieilli à 573K (A2 573K) et un échantillon A1 laminé et vieilli à 873K (A2 873K). De même, la figure 7 montre trois diagrammes de diffraction de rayons X pour un échantillon B1 laminé et non vieilli (B2 CW), un échantillon B1 laminé et vieilli à 573K (B2 573K) et un échantillon B1 laminé et vieilli à 873K (B2 873K).

L'analyse de l'échantillon B2 CW montre la présence de phase β et de martensite α" induite par la déformation. Pour l'échantillon A2 573K, on constate que la transformation inverse de la martensite en austénite est quasiment complète, en maintenant une haute densité de dislocations. Des traces de phase ω et de phase α apparaissent par la décomposition de la phase métastable β. Pour l'échantillon A2 873K, une phase α" est détectée en plus des phases α et β. Des études récentes ont établi que l'effet de la précipitation de la phase α était d'extraire l'oxygène de la matrice de la phase β et d'augmenter la température de début de transformation martensitique Ms. Ceci peut expliquer la présence de phase martensitique après le refroidissement rapide qui suit le traitement thermique à 873K, en particulier pour l'échantillon B2 873K.

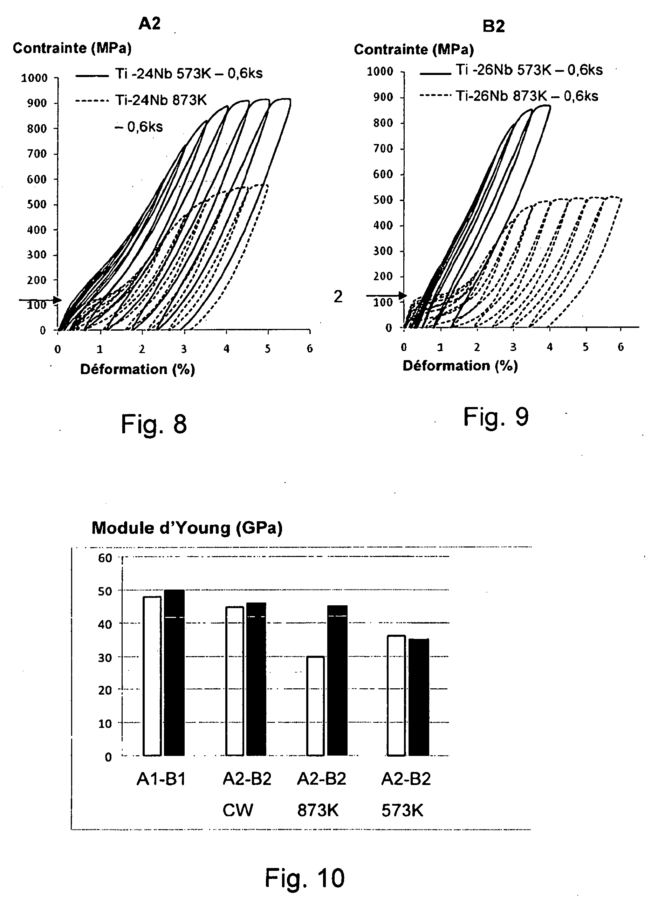

Des essais de caractérisation mécanique ont été conduits en effectuant des cycles de traction-relâchement avec des incréments de déformation constants. Les courbes reliant la déformation à la contrainte sont reportées sur la figure 8 pour les échantillons A2 573K et A2 873K, et sur la figure 9 pour les échantillons B2 573K et B2 873K. On peut noter que ces courbes montrent un comportement mécanique nettement amélioré par rapport aux échantillons A1 et B1 après le recuit de mise en solution et la trempe. En particulier les propriétés super-élastiques sont améliorées : les échantillons A2 573K et B2 873K ont un comportement super-élastique parfait après un allongement imposé de 2% à température ambiante. La précipitation de fines particules de phase α pendant le traitement thermique influence vraisemblablement les propriétés mécaniques par son effet sur la phase β et est responsable de l'augmentation de la contrainte critique de glissement. La contrainte critique de la martensite induite par la contrainte n'est pas affectée pour l'échantillon A2 873K et décroit pour l'échantillon B2 873K, comme le montrent les paliers 1 et 2, en comparaison avec les échantillons A1 et B1. La précipitation de phase α augmente la température Ms, favorisant la formation de martensite induite par la contrainte, ce qui pourrait expliquer l'abaissement de la contrainte critique pour la formation de martensite induite par la contrainte. En outre, la petite taille des grains engendrée par la déformation à froid sévère contribue à l'augmentation de la contrainte critique nécessaire pour la transformation plastique (effet Hall-Petch). Pour les échantillons A2 573K et B2 573K, les courbes de contrainte en fonction de l'allongement montrent un comportement de déformation élastique non linéaire sans double limite élastique. Le comportement de ces alliages est sensiblement différent de ceux traités à 873K, avec en outre une contrainte de glissement considérablement plus haute et une super-élasticité d'environ 2,5%.

Le module d'Young (ou module d'élasticité) est défini comme la pente de la tangente à la courbe allongement-contrainte pour une contrainte nulle. La figure 10 montre la valeur du module d'Young pour les différents échantillons.

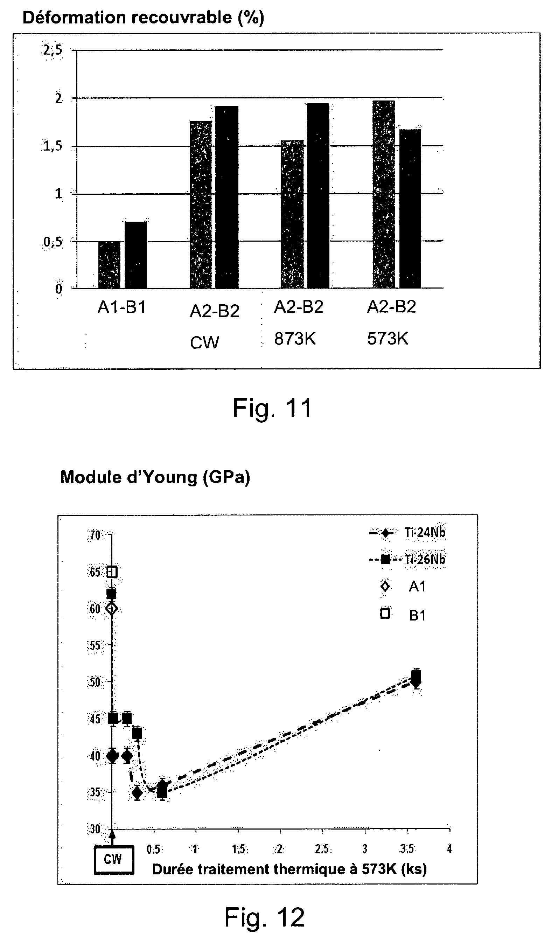

Par ailleurs, la figure 11 montre la déformation recouvrable εᵣ pour les mêmes échantillons.

Pour les échantillons après laminage à froid A2CW et B2CW, le module d'Young est légèrement plus bas que celui des échantillons après la trempe A1 et B1. Après le traitement thermique à 873K, le module d'Young est respectivement de 45 et 30 GPa pour les échantillons A2 873K et B2 873K. Après le traitement thermique à 573K, les deux alliages présentent un module d'Young sensiblement identique de 35 GPa. Il est intéressant de noter que l'abaissement du module d'Young après le traitement thermique ne peut pas être attribué à la fraction volumique de martensite α". Certains auteurs ont indiqué que la décroissance du module d'Young de l'alliage TI-35Nb-4Sn (en proportion massique) par une déformation à froid de 89% serait due à une anisotropie du module d'Young de la martensite α" avec la formation de texture {200}_{α"} [010]_{α"}. Après un traitement thermique à 573K pendant une durée de 20 min, le module d'Young augmente légèrement de 43 GPa à 53 GPa. La phase α en précipité est reconnue pour augmenter le module d'Young. Cependant, la phase α en précipité qui apparaît durant le traitement thermique à 873K pendant 10 min est très petite en taille et en fraction volumique. La baisse du module d'Young, l'amélioration du comportement super-élastique et l'augmentation de la résistance en maintenant un module d'Young faible par la déformation à froid sévère suivie par un traitement thermique de courte durée ne sont pas très bien expliqués par les considérations sur les évolutions de microstructure.

### Exemple 3

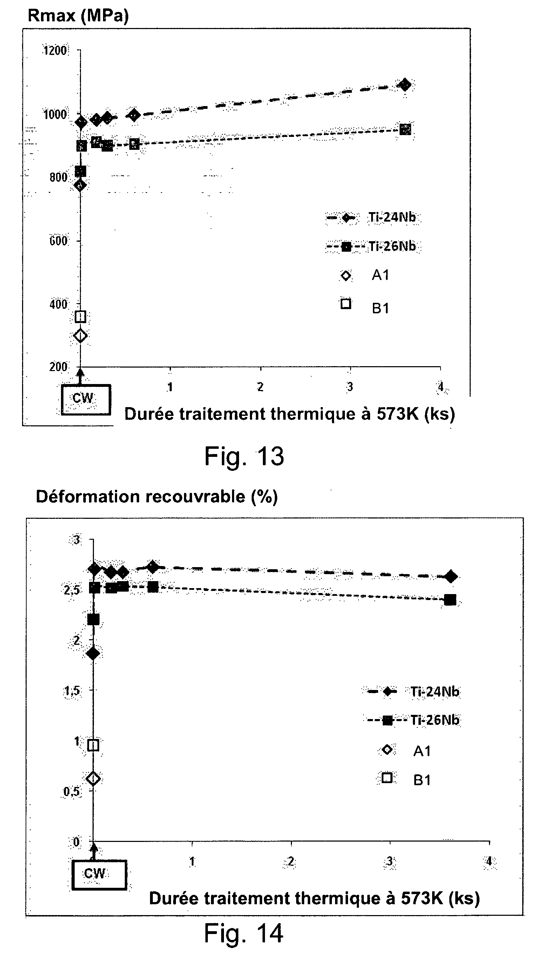

Des échantillons ont été élaborés comme dans l'exemple 2, mais en faisant varier la durée du traitement thermique de vieillissement. Les résultats pour les valeurs respectivement du module d'Young, de la résistance maximale et de la déformation recouvrable pour une déformation imposée de 3% sont présentés sur les figures 12 à 14 en fonction de la durée de vieillissement pour un traitement thermique à 573K. Les exemples 1 et 2 sont également reportés sur ces graphiques par les repères carrés ou en losange blanc. Le premier repère plein sur l'axe des ordonnées correspond à l'état après laminage à froid CW. Le deuxième repère à proximité de l'axe des ordonnées correspond à un traitement de vieillissement d'une durée d'une minute. La figure 12 montre que l'abaissement du module d'Young est obtenu pour une durée courte du traitement de vieillissement, et qu'en le prolongeant, le module d'Young remonte. Seule la remontée du module était connue jusqu'à maintenant. Les figures 13 et 14 montrent que la résistance maximale et la déformation recouvrable sont augmentées très rapidement et sont peu affectées ensuite par l'augmentation de la durée de traitement thermique, même lorsque le module d'Young est bas.

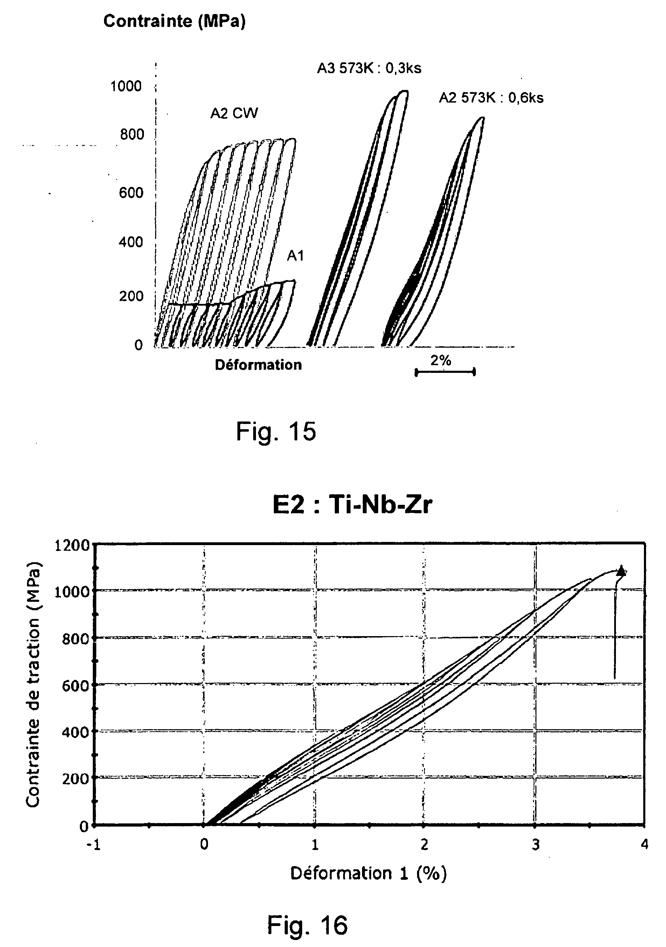

La figure 15 montre les courbes d'essais de traction-relâchement pour les échantillons composés à 24% de niobium. Les courbes des échantillons A1, A2 CW et A2 573K sont à nouveau reproduites, pour comparaison avec celle d'un échantillon A3 573K, ayant suivi un traitement thermique de vieillissement d'une durée de 5 min (0,3 ks).

### Exemple 4

Des échantillons comportant 0,5% d'oxygène ou 0,5% d'azote ont été préparés avec 24% de niobium en masse atomique selon le procédé exposé pour l'exemple 1. Ces composants ont un effet bétagène, c'est-à-dire que la température Ms est abaissée. Ces alliages ont une structure β à température ambiante, comme l'échantillon B1. En comparaison avec l'échantillon B1, dans l'état après le traitement d'homogénéisation et la trempe, la résistance mécanique est nettement améliorée, comme le montrent les résultats dans le tableau 1.

| Echant. | Composition | Structure | Module d'Young (GPa) | Déformation recouvrable (%) | Résistance maximale (MPa) | Allongement (%) |
|---|---|---|---|---|---|---|
| C1 | Ti-24Nb-0.5O | β | 50 | 1,8 | 830 | 22 |
| D1 | Ti-24Nb-0.5N | β | 38 | 2,25 | 680 | 13 |
| B1 | Ti-26Nb- | β | 55 | 1 | 420 | 25 |

### Exemple 5

Un échantillon E2 est réalisé avec une composition de titane, de niobium (20%) et de zirconium (6%, en proportions atomiques) et avec un procédé de traitement thermomécanique comme indiqué dans l'exemple 2, avec une température de traitement de vieillissement de 600°C pendant 10 min. La figure 16 montre la courbe d'essai de traction-déchargement. Un tel échantillon E2 présente un module d'Young de 30 GPa et une résistance maximale de 1100 MPa, avec une déformation recouvrable de 3%.

### Exemple 6

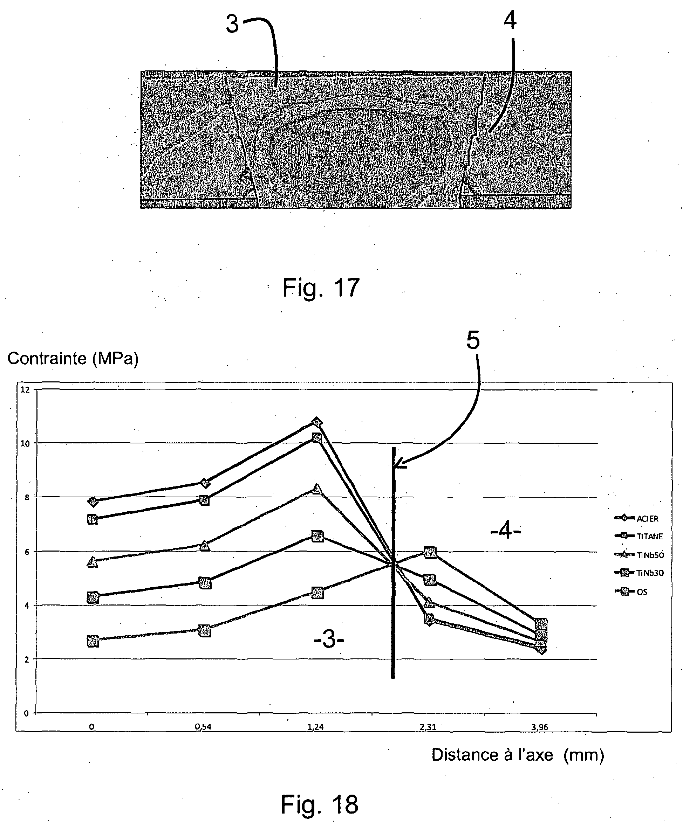

Une simulation numérique de la structure d'un implant dentaire 3 dans de l'os 4 d'un maxillaire a été conduite, en faisant varier le module d'Young de l'implant. Le module d'Young de l'os a été simulé à 20GPa. La figure 17 montre un exemple de répartition des contraintes entre l'implant 3 et l'os 4. La figure 18 trace le niveau de contrainte le long d'un rayon normal à la surface de jonction 3 entre l'implant et l'os, pour différents matériaux d'implant. Les valeurs de modules de Young ont été choisies de la manière suivante :
Acier : 210 GPa
Titane : 110 GPa
TiNb50 : 50 GPa
TiNb30 : 30 GPa
Os : 15 GPa

On constate que le saut de contrainte, de part et d'autre de la surface de jonction 5, est largement diminué avec les alliages au niobium par rapport à l'acier ou au titane pur. Ceci correspond à une diminution de la déviation de contrainte qui se traduit par moins de pertes osseuses autour de l'implant. Même si la valeur du module d'Young est divisée par deux entre l'acier et le titane, la déviation de contrainte reste importante dans les deux cas. Par contre, on constate une différence importante entre un module de 50 GPa et un module de 30 GPa.

## Revendications

1. Procédé de traitement thermomécanique d'un alliage de titane comportant entre 23 et 27% de niobium, entre 0 et 10% de zirconium, et entre 0 et 1% d'oxygène, d'azote et/ou de silicium, les taux étant exprimés en proportion atomique procédé selon lequel on procède aux étapes suivantes :
a) une montée en température d'un échantillon de l'alliage à une température supérieure à 900°C ;
b) une trempe rapide ;
c) une déformation à froid supérieure à une déformation vraie de 3 ;
d) un traitement de vieillissement à une température comprise entre 200 et 600°C, la durée du traitement de vieillissement étant comprise entre 10 secondes et 10 minutes.

2. Procédé selon la revendication 1, dans lequel l'étape a) est précédée d'une étape d'homogénéisation à une température supérieure à 1200 °C pendant au moins 20 heures.

3. Procédé selon la revendication 1, dans lequel, à l'étape c), la déformation est réalisée uniquement en surface d'un échantillon.

4. Alliage de titane comportant entre 23 et 27% de niobium en proportion atomique, entre 0 et 10% de zirconium, et entre 0 et 1% d'oxygène, d'azote et/ou de silicium, **caractérisé en ce qu'**il est obtenu par le procédé selon l'une des revendications 1 à 3 et que son module de Young est inférieur à 45 GPa.

5. Alliage selon la revendication 4, dans lequel le taux de niobium est compris entre 24 et 26% en proportion atomique.

6. Alliage selon la revendication 5, dans lequel le taux de niobium est de 24%, la structure cristallographique étant une phase β avec des traces de martensite α et d'une phase ω après un traitement de vieillissement à 300°C.

7. Alliage selon la revendication 5, dans lequel le taux de niobium est de 24%, la structure cristallographique comportant une phase β, de la martensite α et de la martensite α" après un traitement de vieillissement à 600°C.

8. Alliage selon la revendication 5, dans lequel le taux de niobium est de 26%, la structure cristallographique comportant une phase β et de la martensite α".

9. Alliage selon la revendication 4, dans lequel le taux de zirconium est de 0%.

10. Prothèse implantable dans un corps humain ou animal, **caractérisé en ce qu'**il est réalisé dans un alliage selon la revendication 4.

11. Implant dentaire selon la revendication 10.

## Patentansprüche

1. Verfahren zur thermomechanischen Behandlung einer Titanlegierung, die zwischen 23 und 27% Niob, zwischen 0 und 10% Zirkon und zwischen 0 und 1% Sauerstoff, Stickstoff und/oder Silizium umfasst, wobei die Anteile als Atomverhältnis ausgedrückt sind, wobei nach dem Verfahren die folgenden Schritte durchgeführt werden:
a) eine Erwärmung einer Probe der Legierung auf eine Temperatur über 900 °C;
b) ein schnelles Abschrecken;
c) eine Kaltverformung, die größer ist als eine wahre Dehnung von 3;
d) eine Alterungsbehandlung bei einer Temperatur, die zwischen 200 und 600 °C enthalten ist, wobei die Dauer der Alterungsbehandlung zwischen 10 Sekunden und 10 Minuten enthalten ist.

2. Verfahren nach Anspruch 1, wobei dem Schritt a) ein Homogenisierungsschritt bei einer Temperatur vorausgeht, die während mindestens 20 Stunden höher als 1200 °C ist.

3. Verfahren nach Anspruch 1, wobei im Schritt c) die Verformung einzig auf der Fläche einer Probe ausgeführt wird.

4. Titanlegierung, die im Atomverhältnis zwischen 23 und 27% Niob, zwischen 0 und 10% Zirkon und zwischen 0 und 1% Sauerstoff, Stickstoff und/oder Silizium umfasst, **dadurch gekennzeichnet, dass** sie durch das Verfahren nach einem der Ansprüche 1 bis 3 erhalten wird und dass ihr Elastizitätsmodul niedriger als 45 GPa ist.

5. Legierung nach Anspruch 4, wobei der Niobanteil zwischen 24 und 26% im Atomverhältnis enthalten ist.

6. Legierung nach Anspruch 5, wobei der Niobanteil 24% beträgt, wobei die kristallografische Struktur eine β-Phase mit Spuren von α-Martensit und einer ω-Phase nach einer Alterungsbehandlung bei 300 °C ist.

7. Legierung nach Anspruch 5, wobei der Niobanteil 24% beträgt, wobei die kristallografische Struktur eine β-Phase, α-Martensit und α"-Martensit nach einer Alterungsbehandlung bei 600 °C umfasst.

8. Legierung nach Anspruch 5, wobei der Niobanteil 26% beträgt, wobei die kristallografische Struktur eine β-Phase und α"-Martensit umfasst.

9. Legierung nach Anspruch 4, wobei der Zirkonanteil 0% beträgt.

10. In einen menschlichen oder tierischen Körper implantierbare Prothese, **dadurch gekennzeichnet, dass** sie aus einer Legierung nach Anspruch 4 hergestellt ist.

11. Zahnimplantat nach Anspruch 10.

## Claims

1. Thermomechanical treatment method for a titanium alloy including between 23 and 27% niobium, between 0 and 10% zirconium, and between 0 and 1% oxygen, nitrogen and/or silicon, the ratios being expressed in atomic proportions, method wherein the following steps are performed:
a) an increase in temperature of a sample of the alloy to a temperature higher than 900°C;
b) a fast quench;
c) a cold strain higher than a true strain of 3;
d) an ageing treatment at a temperature comprised between 200 and 600°C, the ageing treatment time being comprised between 10 seconds and 10 minutes.

2. Method according to claim 1, wherein step a) is preceded by a homogenisation step at a temperature higher than 1200°C for at least 20 hours.

3. Method according to claim 1, wherein, at step c), the strain is done only on the surface of a sample.

4. Titanium alloy including between 23 and 27% niobium in atomic proportion, between 0 and 10% zirconium, and between 0 and 1% oxygen, nitrogen and/or silicon, **characterized in that** it is obtained by the method according to one of claims 1 to 3 and that its Young's modulus is lower than 45 GPa.

5. Alloy according to claim 4, wherein the ratio of niobium is comprised between 24% and 26% in atomic proportion.

6. Alloy according to claim 5, wherein the ratio of niobium is 24%, the crystallographic structure being β phase with traces of α martensite and a ω phase after ageing treatment at 300°C.

7. Alloy according to claim 5, wherein the niobium ratio is 24%, the crystallographic structure including a β phase, a martensite and α" martensite after an ageing treatment at 600°C.

8. Alloy according to claim 5, wherein the niobium ratio is 26%, the crystallographic structure including a β phase and α" martensite.

9. Alloy according to claim 4, wherein the zirconium ratio is 0%.

10. Prosthesis implantable in a human or animal body, **characterized in that** it is made from an alloy according to claim 4.

11. Dental implant according to claim 10.
